(19) Europäisches Patentamt
European Patent Office
Office européen des brevets

(11) Veröffentlichungsnummer: **0 014 991**
**B1**

(12) **EUROPÄISCHE PATENTSCHRIFT**

(45) Veröffentlichungstag der Patentschrift: **19.01.83**

(51) Int. Cl.³: **C 12 P 33/06**, C 07 J 1/00, C 07 J 5/00 // C07J7/00

(21) Anmeldenummer: **80100824.4**

(22) Anmeldetag: **19.02.80**

(54) **Mikrobiologisches Verfahren zur Herstellung 7-alpha-hydroxylierter Steroide.**

(30) Priorität: **20.02.79 CH 1675/79**

(43) Veröffentlichungstag der Anmeldung: **03.09.80 Patentblatt 80/18**

(45) Bekanntmachung des Hinweises auf die Patenterteilung: **19.01.83 Patentblatt 83/3**

(84) Benannte Vertragsstaaten: **CH DE FR GB IT**

(56) Entgegenhaltungen:
**EP-A-0 001 102**
**US-A-2 960 513**
**US-A-2 962 512**
**CHEMICAL ABSTRACTS, Band 75, Nr. 11, 13. September 1971, Zusammenfassung Nr. 72841w, Seiet 142 Columbus, Ohio, US, H. J. BRODIE et al.: »Microbiological hydroxylation of estr-4-ene-3,17-dione«**

(73) Patentinhaber: **F. HOFFMANN-LA ROCHE & CO. Aktiengesellschaft, CH-4002 Basel (CH)**

(72) Erfinder: **Fujiwara, Mitsuhiko, 1059-7 Fueta, Kamakura-shi Kanagawa-ken (JP)**
Erfinder: **Fujiwara, Akiko, 1059-7 Fueta, Kamakura-shi Kanagawa-ken (JP)**
Erfinder: **Miyamoto, Chikara, 505 Marumo Building 250 Kuden-cho Totsuka-ku, Yokohama-shi Kanagawa-ken (JP)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

**EP 0 014 991 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Mikrobiologisches Verfahren zur Herstellung von 7α-hydroxylierten Steroiden

Die vorliegende Erfindung betrifft ein mikrobiologisches Verfahren zur Herstellung 7α-hydroxylierter Steroide durch Fermentierung 7-unsubstituierter Steroide der Pregnan- oder Androstanreihe mit Mikroorganismen des Genus Botryodiplodia.

Die mikrobielle 7α-Hydroxylierung von Steroiden ist bekannt. Beispielsweise wird in Canadian Journal of Microbiology, Vol. 13, Seiten 1271–1281 (1967) die 7α-Hydroxylierung von Steroiden mittels Mucor griseocyanus beschrieben. Überraschend wurde nun gefunden, daß eine 7α-Hydroxylierung von Steroiden mit Mikroorganismen des Genus Botryodiplodia, welche gänzlich verschieden von Mucor sp. sind, ausgeführt werden kann.

Erfindungsgemäß verwendet man als Ausgangsverbindungen Dehydroepiandrosteron, Pregnenolen oder Steroide der Formel

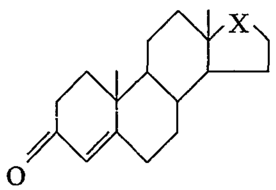

(I)

worin X eine Gruppe

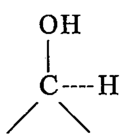

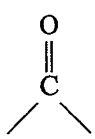

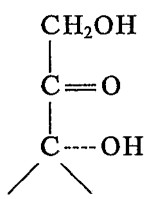

darstellt.

Bei Verwendung von Verbindungen der Formel I als Ausgangsmaterial erhält man mittels des erfindungsgemäßen Verfahrens Verbindungen der Formel II

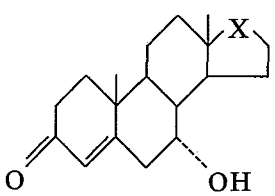

(II)

worin X die obige Bedeutung besitzt.

Bei der erfindungsgemäßen Fermentation von Dehydroepiandrosteron erhält man 7α-Hydroxy-4-androsten-3,17-dion und 7α,17β-Dihydroxy-4-androsten-3-on; bei der Fermentation von Pregnenolon das 7α-Hydroxy-progesteron.

Erfindungsgemäß können alle Stämme des Genus Botryodiplodia, die zur 7α-Hydroxylierung von Steroiden, insbesondere solchen der Formel I, Pregnenolon und Dehydroepiandrosteron befähigt sind, verwendet werden. Bevorzugte Stämme sind Botryodiplodia theobromae IFO 6469 und Botryodiplodia malorum CBS 134.50.

Die Mikroorganismen können als Kultur, in Form des Mycels oder in aufbereiteter Form, z. B. in Form eines aus der Kulturlösung oder dem Mycel in an sich bekannter Weise hergestellten Enzymextrakts verwendet werden. Die Kulturlösung kann durch Beimpfen eines geeigneten Mediums mit dem Mikroorganismus hergestellt werden. Geeignete Medien sind solche, die Kohlenstoffquellen, Stickstoffquellen, anorganische Salze u. a. für das Wachstum des Mikroorganismus geeignete Nährstoffe enthalten. Als Kohlenstoffquelle kommen beispielsweise Glukose, Saccharose, Dextrin, Mannose, Stärke, Laktose und Glycerin in Betracht; Stickstoffquellen sind beispielsweise stickstoffhaltige, organische Substanzen wie Pepton, Fleischextrakt, Hefeextrakt, Maisquellwasser und Kasein, oder stickstoffhaltige, anorganische Verbindungen wie Nitrate und anorganische Ammoniumsalze. Als anorganische Salze sind Phosphate oder Natrium-, Kalium-, Magnesium-, Mangan-, Eisen- und Kupfersalze zu erwähnen.

Die Züchtung des Mikroorganismus kann als Submerskultur, als Schüttelkultur oder als stationäre Kultur durchgeführt werden; vorzugsweise wird der Mikroorganismus unter aeroben Bedingungen kultiviert.

Das erfindungsgemäße Verfahren wird zweckmäßig so durchgeführt, daß die zu hydroxylierende Verbindung der allgemeinen Formel I bzw. Pregnenolon oder Dehydroepiandrosteron als Substrat zu der angezüchteten Kulturlösung gegeben wird. Die Konzentration des Substrates beträgt zweckmäßig 0,1 g pro Liter bis 20 g pro Liter. Die erfindungsgemäße Hydroxylierung kann durch Fortsetzung der Kultivierung des Mikroorganismus unter den obenerwähnten Bedingungen durchgeführt werden. Die Reaktionszeit kann in Abhängigkeit von Spezies und Stamm des verwendeten Mikroorganismus, von der Zusammensetzung des Kulturmediums, vom verwendeten Substrat und dessen Konzentration variieren. Im allgemeinen genügt eine Fermentationszeit von 1–10 Tagen. Die Reaktionstemperatur liegt allgemein zwischen 20 und 30°C, zweckmäßig arbeitet man bei einem pH von 4–9.

Das Substrat kann der Kultur des Mikroorganismus auch während der Anzüchtung oder dem Kulturmedium vor der Sterilisation oder der Beimpfung zugesetzt werden.

Die erfindungsgemäße Hydroxylierung kann auch mit dem aus der Kulturlösung isolierten Mycel des Mikroorganismus oder mit einem aus der Kulturlösung oder dem Mycel in an sich bekannter Weise hergestellten Enzymextrakt durchgeführt werden. In diesem Falle kann die 7α-Hydroxylierung zweckmäßig in wäßriger Lösung, z. B. einer Pufferlösung, in physiologischer Salzlösung, in frischer Nährlösung oder in Wasser durchgeführt werden.

Das Substrat (das zu hydroxylierende Steroid)

kann in fester Form oder als Lösung in einem hydrophilen Lösungsmittel wie Aceton, Dimethylsulfoxid, Methanol, Äthanol, Äthylenglykol, Propylenglykol oder Dioxan zugesetzt werden. Man kann auch einer wäßrigen Suspension des Substrats ein oberflächenaktives Mittel oder ein Dispersionsmittel zusetzen, oder das Substrat durch Behandlung mit Ultraschall emulgieren.

Mittels des erfindungsgemäßen Verfahrens erhält man aus 17α,21-Dihydroxy-4-pregnen-3,20-dion und 17β-Hydroxy-4-androsten-3-on das entsprechende 7α-hydroxylierte Steroid, d. h. 7α,17α,21-Trihydroxy-4-pregnen-3,20-dion bzw. 7α,17β-Dihydroxy-4-androsten-3-on.

Das Fermentationsprodukt kann aus dem Reaktionsgemisch in an sich bekannter Weise isoliert werden, beispielsweise durch Solventextraktion mit einem organischen Lösungsmittel, das mit Wasser nicht mischbar ist, wie Chloroform, Methylenchlorid oder Methylacetat oder durch Chromatographie auf Trägern wie Aluminiumoxyd, Silicagel oder Cellulose. Das Fermentationsprodukt kann auch durch Umkristallisation, z. B. aus Äthylacetat, Benzol oder Aceton gereinigt werden.

Bei Verwendung von 4-Androsten-3,17-dion als Ausgangsmaterial erhält man ein Gemisch von 7α-Hydroxy-4-androsten-3,17-dion und 7α,17β-Dihydroxy-4-androsten-3-on. Dieses Gemisch kann leicht durch Chromatographie getrennt werden, wobei man die Polarität des Elutionsmittels variiert.

Die Verfahrensprodukte sind bekannte Verbindungen, die als Zwischenprodukte zur Herstellung synthetischer Hormone, Cholsäure und verschiedener Pharmazeutika von Wert sind. Die folgenden Beispiele illustrieren das erfindungsgemäße Verfahren. Die Temperaturen sind in Celsiusgraden angegeben.

## Beispiel 1

Ein Kulturmedium mit 1% Maisquellwasser und 1% Glukose wurde auf pH 6,5 eingestellt. 100 ml dieses Mediums wurden in einer 500 ml-Flasche mit Papierstopfen 15 Minuten bei 120° sterilisiert. Nach Abkühlen wurde mit dem Mycel einer zwei Wochen alten Malzextrakt-Agarkultur von Botryodiplodia theobromae IFO 6469 beimpft. Die Kultur wurde dann bei 26,5° mit 180 Bewegungen pro Minute rotierend geschüttelt. Nach 3 Tagen wurden 300 mg 4-Androsten-3,17-dion, die durch 10 Minuten Beschallung in 3 ml 0,1%iger Tween 30-Lösung emulgiert worden waren, zugesetzt. Die Inkubation wurde dann 6 Tage fortgesetzt, danach wurde die Kulturflüssigkeit abfiltriert und das Mycel mit Wasser gewaschen, so daß das Endvolumen von Filtrat und Waschwasser 110 ml betrug.

110 ml Kulturfiltrat wurden dreimal mit je 100 ml Äthylacetat extrahiert. Die vereinigten Extrakte wurden über Natriumsulfat getrocknet und unter vermindertem Druck auf 5 ml eingedampft. Das Konzentrat wurde an Kieselsäure (Mallinckrodt) unter Verwendung von Chloroform-Aceton als Elutionsmittel chromatographiert. Mit Chloroform-Aceton (19 : 1) wurde 7α-Hydroxy-4-androsten-3,17-dion und mit Chloroform-Aceton (15 : 3) 7α,17β-Dihydroxy-4-androsten-3-on eluiert. Die zusammengehörenden Fraktionen wurden gesammelt und aus Aceton umkristallisiert. Man erhielt 80,7 mg 7α-Hydroxy-4-androsten-3,17-dion, Schmelzpunkt 254,5 – 256,5° und 46,0 mg 7α,17β-Dihydroxy-4-androsten-3-on, Schmelzpunkt 191 – 193°.

## Beispiel 2

Ein Fermentationsmedium enthaltend 1% Laktose, 3% Bacto-liver (Difco), 0,1% $KH_2PO_4$ und 0,05% KCl wurde auf pH 6,3 gestellt und 15 Minuten bei 120°C sterilisiert. Der Nährboden wurden in zehn 100 ml-Portionen mit dem Mycel einer zwei Wochen alten Malzextrakt-Schrägagarkultur von Botryodiplodia theobromae IFO 6469 beimpft. Die Kulturen wurden dann bei 26,5° mit 180 Bewegungen pro Minute auf der Roationsmaschine geschüttelt. Nach 22 Stunden wurden je 50 mg 17α,21-Dihydroxy-4-pregnen-3,20-dion (vorher durch 10 Minuten Beschallung in 1 ml 0,1% Tween 80 emulgiert) zugesetzt, so daß die Konzentration an Steroid 0,5 mg/ml Fermentationslösung betrug. Die Kulturen wurden dann weitere 92 Stunden inkubiert, danach gesammelt, filtriert und mit Wasser gewaschen, so daß aus 1000 ml Nährlösung ein Totalvolumen von 1100 ml erhalten wurde.

Die so erhaltenen 1100 ml Kulturlösung wurden mit Äthylacetat extrahiert und unter vermindertem Druck auf ein kleines Volumen eingeengt. Der Rückstand wurde an Kieselsäure unter Verwendung von Chloroform-Aceton als Elutionsmittel chromatographiert. Die zusammengehörenden Fraktionen wurden gesammelt und aus Äthylacetat kristallisiert. Man erhielt 188 mg 7α,17α,21-Trihydroxy-4-pregnen-3,20-dion, Schmelzpunkt 221,5 – 223,5°.

## Beispiel 3

100 ml eines Nährmediums, enthaltend 1% Maisquellwasser und 1% Glukose, wurden auf pH 6,5 gestellt und nach Sterilisation mit dem Mycel von Botryodiplodia theobromae IFO 6469 beimpft. Nach Inkubation bei 26,5° auf der Rotationsschüttelmaschine während 22 Stunden wurden 100 mg 17β-Hydroxy-4-androsten-3-on in 1 ml Dimethylsulfoxid zugegeben und die Inkubation wurde bei 26,5° weitere 8 Tage fortgeführt. Danach wurde die Fermentationslösung filtriert, das Filtrat mit Äthylacetat extrahiert und auf ein kleines Volumen unter vermindertem Druck eingeengt. Das Konzentrat wurde an Kieselsäure mit Chloroform-Aceton chromatographiert. Man erhielt 17,9 mg 7α,17β-Dihydroxy-4-androsten-3-on.

Beispiel 4

100 ml eines Nährmediums, enthaltend 2% Saccharose, 1% S-3 meat (Ajinomoto Co.), 1% Pepton und 0,5% $KH_2PO_4$ wurde auf pH 6,5 eingestellt, sterilisiert und mit Mycel von Botryodiplodia malorum CBS 134.50 beimpft. Nach zweitägiger Inkubation bei 26,5° auf der Rotationsschüttelmaschine wurden 50 mg 17α,21-Dihydroxy-4-pregnen-3,20-dion in 1 ml Dimethylsulfoxid zugesetzt, und die Fermentierung wurde weitere 6 Tage bei 26,5° fortgeführt. Danach wurde die Fermentationslösung gesammelt und wie in den vorangehenden Beispielen aufgearbeitet. Man erhielt 8,5 mg 7α,17α,21-Trihydroxy-4-pregnen-3,20-dion.

Beispiel 5

100 ml eines Fermentationsmediums, das 1% Glukose und 1% Maisquellwasser enthielt und einen pH von 6,5 aufwies, wurde 15 Minuten bei 120° sterilisiert. Das Medium wurde mit dem Mycel einer zwei Wochen alten Malzextrakt-Agarkultur von Botryodiplodia theobromae IFO 6469 beimpft. Nachdem die Kultur zwei Tage lang auf einer Rotationsschüttelmaschine bei 26,5° inkubiert worden war, wurden 100 ml 3β-Hydroxy-5-androsten-17-on, gelöst in 1 ml Dimethylsulfoxid, zugesetzt, und die Inkubation wurde drei weitere Tage fortgesetzt.

Das Kulturfiltrat wurde dann dreimal mit je 100 ml Äthylacetat extrahiert, die Extrakte über Natriumsulfat getrocknet und unter vermindertem Druck auf ein kleines Volumen eingeengt. Das Konzentrat wurde an einer Kieselsäure-(Mallinckrodt)Säule mit Chloroform-Aceton chromatographiert. Mit einem Gemisch von Chloroform-Aceton (19 : 1) wurde 7α-Hydroxy-4-androsten-3,17-dion und mit Chloroform-Aceton (15 : 3) 7α,17β-Dihydroxy-4-androsten-3-on eluiert. Die zusammengehörigen Fraktionen wurden vereinigt und aus Aceton kristallisiert und lieferten 20 mg 7α-Hydroxy-4-androsten-3,17-dion, Schmelzpunkt 254–255°C, und 10,5 mg 7α,17β-Dihydroxy-4-androsten-3-on, Schmelzpunkt 191°.

Beispiel 6

In Analogie zu Beispiel 5 wurden 50 mg 3β-Hydroxy-5-pregnen-20-on zwei Tage lang fermentiert. Man erhielt 10,5 mg 7α-Hydroxy-4-pregnen-3,20-dion.

**Patentansprüche**

1. Mikrobiologisches Verfahren zur Herstellung 7α-hydroxylierter Steroide, dadurch gekennzeichnet, daß man Dehydroepiandrosteron, Pregnenolon oder ein Steroid der Formel

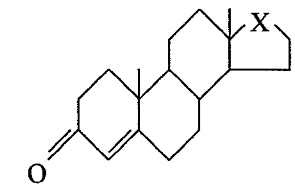

(I)

worin X eine Gruppe

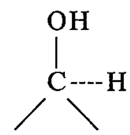

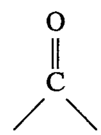

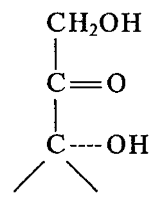

darstellt,
mit Mikroorganismen des Genus Botryodiplodia, die zur 7α-Hydroxylierung von Steroiden befähigt sind, fermentiert.

2. Verfahren nach Anspruch 1, dadurch gekennzeichnet, daß man als Mikroorganismus Botryodiplodia theobromae IFO 6469 verwendet.

3. Verfahren nach Anspruch 1, dadurch gekennzeichnet, daß man als Mikroorganismus Botryodiplodia malorum CBS 134.50 verwendet.

**Claims**

1. Microbiological process for the manufacture of 7α-hydroxylated steroids, characterized by fermenting dehydroepiandrosterone, pregnenolone or a steroid of the formula

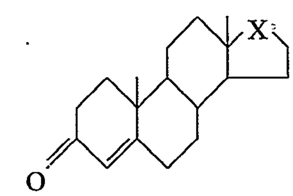

(I)

wherein X represents a group

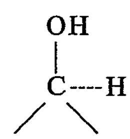

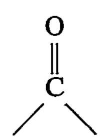

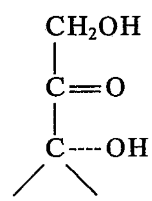

with microorganisms of the genus Botryodiplodia which are capable of 7α-hydroxylating steroids.

2. Process according to claim 1, characterized in that Botryodiplodia theobromae IFO 6469 is used as the microorganism.

3. Process according to claim 1, characterized in that Botryodiplodia malorum CBS 134.50 is used as the microorganism.

## Revendications

1. Procédé microbiologique de préparation de stéroïdes 7α-hydroxylés, caractérisé en ce qu'on fait fermenter de la déshydroépiandrostérone, de la pregnénolone ou un stéroïde de formule

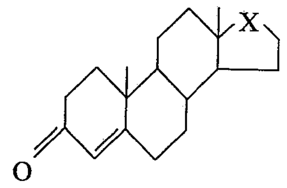

(I)

où X représente un groupe

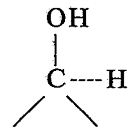

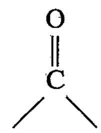

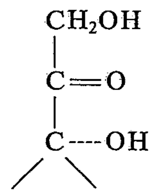

avec des microorganismes du genre Botryodiplodia qui sont aptes à la 7α-hydroxylation des stéroïdes.

2. Procédé selon la revendication 1, caractérisé en ce qu'on utilise comme microorganisme Botryodiplodia theobromae IFO 6469.

3. Procédé selon la revendication 1, caractérisé en ce qu'on utilise comme microorganisme Botryodiplodia malorum CBS 134.50.